Europäisches Patentamt

European Patent Office

Office européen des brevets

(19)

(11) Publication number: **0 086 475**
**A2**

(12) # EUROPEAN PATENT APPLICATION

(21) Application number: **83101340.4**

(51) Int. Cl.³: **A 61 K 35/12**

(22) Date of filing: **11.02.83**

(30) Priority: **16.02.82 JP 23977/82**

(43) Date of publication of application: **24.08.83**
**Bulletin 83/34**

(84) Designated Contracting States: **DE FR GB IT**

(71) Applicant: **NIPPON SHINYAKU COMPANY, LIMITED, 14,**
**Kisshoin Nishinosho Monguchicho, Minami-ku Kyoto-shi**
**Kyoto (JP)**

(72) Inventor: **Enomoto, Hiroshi, 26-3-7-707 Babakemba**
**Hashiri, Nagaokakyo-shi Kyoto-fu (JP)**
Inventor: **Ozaki, Masakuni, 7-25 Mitani Terada, Joyo-shi**
**Kyoto-fu (JP)**
Inventor: **Watanabe, Hisao, 1-6-353-9 Ninomaru-cho,**
**Mukaijima Fushimi-ku Kyoto (JP)**
Inventor: **Matsuda, Masato, 501 Abuno Daini**
**mans. 371 4-chome Tsukahara, Taktasuki-shi**
**Osaka-fu 569 (JP)**

(74) Representative: **Wey, Hans-Heinrich, Dipl.-Ing. et al,**
**Patentanwälte Müller-Börner Wey & Körner**
**Widenmayerstrasse 49, D-8000 München 22 (DE)**

(54) **A method of manufacturing anti-tumor substances.**

(57)     An anti-tumor substance is produced by a method, in which immunity donator is administered to animals intraperitoneally, then lipopolysaccharide derived from gram-negative bacillus administered thereto, large amount of physiological saline solution injected into abdominal cavity simultaneously, then the physiological saline solution is recovered therefrom and purified.

EP 0 086 475 A2

COMPLETE DOCUMENT

A Method of Manufacturing Anti-Tumor Substances

The present invention relates to a method of manufacturing anti-tumor substances and, more particularly, it relates to a method of recovering humoral factor with an anti-tumor activity of high purity in high yield by the utilization of animal cells derived from monocytes.

The above factor exhibits the same action as TNF (Tumor necrosis factor) found by Dr. Old of Memorial Sloan Kettering Cancer Center of U. S. A.

About the therapeutic methods for cancer, surgical therapy, radioactive therapy, chemotherapy and immunotherapy have been mostly conducted today. However any of them cannot be said to be a therapy for complete recovery and, particularly in chemotherapy, there is a disadvantage of much injury to normal cells.

In 1975, Dr. Old et al of the Memorial Sloan Kettering Cancer Center, U. S. A., reported about TNF which is related to glycoprotein and which does not damage normal cells at all but selectively attack cancer cells (cf. E. A. Carswell, et al: Proc. Nat. Acad. Sci. USA, volume 72, page 3666, 1975). According to this report, BCG or the like is administered to vain of mice and, after one to two weeks, lipopolysaccharide (LPS) which is bacterial endo toxin derived from Gram negative bacillus is administered and, one to two hours thereafter, serum is isolated therefrom to give desired anti-tumor factor, i.e. crude solution of TNF.

However, mice which are given LPS cause endotoxin shock and, therefore, the amount of serum taken is small and, in addition, it contains very many impurities since the crude solution is serum and it is troublesome and difficult to remove such impurities. Thus, loss of desired substance during the purification step is large. This is the reason why TNF has not be obtained in large quantities though it has been well known that TNF is very useful anti-cancer agent.

The present inventors have conducted extensive studies in order to obtain the anti-tumor substance which are useful in the cancer therapy in large quantities and in purer form containing less impurities and accomplished the present invention.

The present invention will be described in detail as hereunder.

Thus, inactivated anaerobic Corynebacterium, BCG, Listeria monocytogenes and the like is administered to such animals as mice, rats, rabbits, and the like intraperitoneally and, one to two weeks thereafter, lipopolysaccharide (LPS) which is a endotoxin derived from Gram negative bacillus intravenously or intraperitoneally and, at the same time, large amount of physiological saline solution is injected into abdominal cavity and, two to four hours later, the animals are treated with needles or laparotomized to recover physiological saline solution containing useful desired substance.

The resulting crude solution is subjected to ultracentrifugation, ion exchange resin adsorption, gel filtration, electrophoresis, and the like so that it·can be purified comparatively easily.

In carrying out the present invention, any animal which is sensitive to LPS which is an endotoxin derived from Gram negative bacillus may be used. Examples of such animals are mice, rats, rabbits, and goats.

The present invention will now be described in some more detail. Thus, there is no changes in activity whenever LPS is given during seventh and tenth days after administration of inactivated anaerobic Corynebacterium. Any lipopolysaccharide derived from Gram negative bacillus may be used as LPS and, therefore, all of Escherichia coli, Salmonella, Serratia, Shigella, etc can give the same active substances. As to doses to animals, 0,5 to 50 gammas per mouse by intraperitoneal injection or 10 to 30 gammas per mouse by intravenous injection may afford the same active substances. The time from administration of LPS to isolation of desired substance will suitably be two to four hours; when it exceeds six hours, the activity will disappear.

Anti-tumor activity of desired substances obtained by the present invention can be confirmed by measuring cell injuries using cancer cells and normal cells as target cells. It has also been confirmed that normal cells are not injured. Cancer cells used as target cells here are fibroblast derived from mice (L-929; NCTC-Clone 929). They are inoculated to Eagle's MEM medium (Nissui) for tissue culture to which is added 10% fetal calf serum (Gibco) to make the cell numbers 400,000 per milliliter, divided into multi-well plates (Limbro) with 24 holes for tissue culture, mixed with the desired substance diluted with the above medium, cultured at 37°C in 5% $CO_2$-95% air and, after 72 hours, the state of cell injury is observed.

As to normal cells, healthy mice are sacrificed, kidney is isolated therefrom under aseptic conditions, cut with scissors, digested with 0.25% trypsin with stirring at 4°C, centrifuged at 1000 rotations per minute for five minutes, the collected cells are inoculated to kanamycin-added tissue culture medium RPMI-1640 (Nissui) (to which is added 10% fetal calf serum of Gibco) to make the cell numbers 400,000/ml, and cell injury is observed by the same operation as in cancer cells.

Cell injury is observed as follows. Thus, culture is conducted at 37°C in 5% $CO_2$-95% air for 72 hours, the medium is discarded, the well to which viable cells are adhered as mono-layer is washed with PBS (phosphate bufferred physiological saline solution), 0.036% Neutral Red PBS solution is added thereto, allowed to stand in 5% $CO_2$-95% air at 37°C and, after one hour, it is taken out and the well is well washed with PBS, extracted with an equi-volume mixture of 0.1M $NaH_2PO_4$ solution and 99.5% ethanol whereupon Neutral Red incorporated in viable cells is taken out, and absorbancy at 540 nm wave length is measured. The result is given in Table 1 wherefrom it is apparent that the physiological saline solution containing the desired substance injures cancer cells significantly with a probability of 1 to 2%, as compared with the control. Though not given in the table, there is no effect on normal cells at all.

As a result, it has been confirmed that the crude aimed substances produced by a method of intravenous injection of LPS or by a method of intraperitoneal injection of LPS can sufficiently injures cancer cells specifically.

The physiological saline solution containing the aimed substances is subjected to ultracentrifugation, the supernatant liquid obtained is collected, treated with DEAE-Sephadex or CM-Sephadex, subjected to a gel filtration with Sephadex G-100 to collect active fractions and subjected to a polyacrylamide gel electrophoresis to give pure aimed product which does not contain LPS.

This product in a pure form as purified the crude one obtained from mice is a sugar protein having the following properties of 40,000 molecular weight as measured by gel filtration using Sephadex G-200, 55,000 molecular weight as measured by SDS polyacrylamide electrophoresis, electrophoresed from a region of alpha-globulin to a region of albumin by polyacrylamide electrophoresis, stable at 56°C for 30 minutes, stable at pH 5 to 10, having isoelectric point of 5.0 to 5.2, having no affinity with lectins (such as concanavalin A and phytohemagglutinin), having no affinity with various dyestuffs in Dye-matrix kit of Amicon Company such as Cibacron Blue F3G-A, and containing sialic acid therein.

In order to afford large quantities of pure product from crude substances, the following operations are conducted.

The pure aimed product is subjected to subcutaneous injection for several times to rabbits together with Freund's adjvant (complete) (Nakarai Kagaku), then immunized by intravenous injection, blood is taken tentatively, antibody titer is measured using a disappearance of cytocidal effect of the aimed product against L-929 cells as a marker and, when the antibody titer is sufficiently increased, blood is taken out to give antiserum, then passed through a column of CNBr activated sepharose 4B (Pharmacia) with which the above pure aimed product is combined whereupon all antibodies except those for the aimed product are removed, and aimed antibody is obtained.

This is isolated with 50% ammonium sulfate, precipitates are collected, purified with protein A-sepharose to collect IgG fraction, the fraction is combined with CNBr activated cepharose 4B, and filled in a column. Crude aimed product is passed through the column to conduct affinity chromatography so that pure aimed product is obtained. By carrying out the above method, pure aimed product can be obtained from crude substance by simple operations in high yields.

Meth-A (which is a fibrosarcoma) induced by 3-methylchoranthren is subcutaneously transplanted to mice (1,000,000 cells/mouse) and, when tumor becomes 5 to 7 mm diameter after seven days, the above aimed product is administered intravenously to mice of BALB/c strain. It has been found that, within 24 hours after administration, the cancer tumor shows hemorrhagic necrosis.

The above result is evaluated in accordance with the classification by Carswell, Old, et al. on the strength of the activity as is given in Table 2. Here again, only cancer tumor shows hemorrhagic necrosis but there is no effect on tissue of the host mice.

When LPS is administered intravenously, the aimed product shows cell injury against cancer and, in addition, due to non contamination of LPS, purification will be very easy and advantageous. When LPS is administered intraperitoneally, it is not necessary to sacrifice the animal if crude aimed substance is recovered from the animal by inserting a needle of sterilized syringe under sterilized conditions and, after several term, they can be repeatedly used in the manufacture of crude aimed substance and, therefore, very advantageous.

Processes of obtaining aimed product in large quantities and in a state of less impurities are given in the following examples.

Example 1.

Cultured Corynebacterium parvum are inactivated by heating or by formaline treatment and lyophilized or by re-suspended in physiological saline solution to give inactivated cells of anaeronic Corynebacteria. One milligram/mouse of them (as dry weight) are administered to ICR-strain female mice intraperitoneally. After 8 days, 5 ml of physiological saline solution containing 1 gamma/ml of LPS (Difco) of Escherichia coli (which is a kind of endotoxins derived from Gram negative bacillus) is injected intraperitoneally to mice and, after 2 hours, the mice are slaughtered by decapitation, laparotomized immediately, the physiological saline solution containing the aimed product is carefully taken out with a pipette so as not to injure inner organs and other tissues, and recovered.

The resulting physiological saline solution is placed in a centrifugal tube, centrifuged at 4°C for ten minutes at 3000 rotations per minute to remove solids such as abdominal cells, and the supernatant fluid is obtained as crude aimed product solution.

Example 2.

Inactivated mycelia of anaerobic Corynebacteria which are the same as those in Example 1 are given intreperitoneally to ICR strain mice at the concentration of 1 mg (dry weight) per mouse and, after eight days, endotoxin LPS derived from the same Gram negative bacillus as in Example 1 is adminstered intravenously, after dissolving in physiological saline solution, to mice with a dose of 10 gammas/0.2 ml/mouse and, immediately thereafter, 5 ml of physiological saline solution is injected intraperitoneally. After 2 hours, the mice are slaughtered by decapitation and laparotomized immediately thereafter. If and when mice are dead due to endotoxin shock during the operation, they are laparotomized at that stage and then the same operations as in Example 1 are conducted to give crude aimed product.

Example 3.

Mice are administered, the same as in Example 1, with 1 mg (dry weight) per mouse of anaerobic Corynebacteria inactivated mycelia and, after 8 days, physiological saline solution containing endotoxin LPS derived from Gram negative bacillus intraperitoneally the same as in Example 1. After two hours, the mice are placed on a board on their backs, immobilized, hairs on the belly surface are cut, sterilized with 70% alcohol, then sterilized syringe (10 ml volume; needles are 22G 1 1/2 made by Thermo Co) is penetrate therein, and sucked carefully not to damage tissues to recover crude aimed substances.

The animals used here are bred for one month with careful attention on infection at scars and the above operations of intraperitoneal injection of inactivated mycelia of anaerobic Corynebacteria and thereafter are repeated so that crude aimed substances are recovered.

~12~

Table 1. Cell injuring effect of crude aimed substances
in cell culture system against L-929 cancer cells
(Crude aimed substances are 20 times diluted in
each group and subjected to tests)

(1) When LPS is administered intraperitoneally

2 hrs  4 hrs  6 hrs  cont.
(Elapse of time from LPS administration to collection)

(2) When LPS is administered intravenously

2 hrs  4 hrs  6 hrs  cont.
(Elapse of time from LPS administration to collection)

Table 2. Hemorrhagic necrosis effect of tumor by purified
aimed product against Meth A cancer transplanted
to BALB/c mice

| Route of Adminis-<br>tration of LPS | Elapse of Time from LPS Administration to Collection | | | |
|---|---|---|---|---|
| | 2 hours | 4 hours | 6 hours | Control |
| Intraperitoneal | +++ | + | - | - |
| Intravenous | ++ | *** | *** | - |

***: Collection impossible due
to death of mice

Evaluation as mentioned above was conducted in accordance
with a classification in Fig. 1 in page 3667 in a report by
E. A. Carswell, et al (Proc. Nat. Acad. Sci. USA, volume 72,
1975).

(END)

We Claim:

(1) A method of manufacturing anti-tumor substance, characterized in that, immunity donator is administered to animals intraperitoneally, then lipopolysaccharide derived from Gram-negative bacillus administered thereto, large amount of physiological saline solution injected into abdominal cavity simultaneously, then the physiological saline solution is recovered therefrom and purified.

(2) A method of Claim 1 in which the animal is mouse, rabbit or rat.

(3) A method of Claim 1 or 2 in which the immunity donator is inactivated mycelia of Listeria monocytogenes, BCG or anaerobic Corynebacteria.

(4) A method of Claims 1 to 3 in which the lipopolysaccharide derived from Gram negative bacillus is administered intravenously.

(5) A method of Claims 1 to 3 in which the lipopolysaccharide. derived from Gram negative bacillus is administered intraperitoneally.